# FASCICULE DE BREVET EUROPEEN

(11) **EP 2 523 967 B1**
(45) Date de publication et mention de la délivrance du brevet: **18.12.2013**
(21) Numéro de dépôt: 11703722.6
(22) Date de dépôt: 13.01.2011
(51) Int. Cl.: C07K 7/06, C07K 7/08

(54) **NOUVEAUX PEPTIDES ANTI-AGE MODULATEURS DE LA SURVIVINE ET COMPOSITIONS LES COMPRENANT**
NEUE ANTI-AGEING-PEPTIDE ZUR SURVIVIN-MODULIERUNG UND ZUSAMMENSETZUNGEN DAMIT
NOVEL ANTI-AGEING PEPTIDES MODULATING SURVIVIN AND COMPOSITIONS INCLUDING SAME

(30) Priorité: 14.01.2010 FR 1000134
(43) Date de publication de la demande: 21.11.2012
(73) Titulaire: ISP Investments Inc., Wilmington, DE 19805 (US)
(72) Inventeur: DAL FARRA, Claude, Kerhonkson NY 12446 (US); DOMLOGE, Nouha, F-06560 Valbonne (FR); BOTTO, Jean-Marie, F-06560 Valbonne (FR)
(74) Mandataire: Macquet, Christophe
(86) Numéro de dépôt international: PCT/FR2011/000017
(87) Numéro de publication internationale: WO 2011/086296

(56) Documents cités:
- WO-A2-2007/039192
- MARCONI ALESSANDRA ET AL: "Survivin identifies keratinocyte stem cells and is downregulated by anti-beta 1 integrin during anoikis", STEM CELLS (MIAMISBURG), vol. 25, no. 1, janvier 2007 (2007-01), pages 149-155, XP002587551, ISSN: 1066-5099
- XU ZHENJIE ET AL: "INCENP-aurora B interactions modulate kinase activity and chromosome passenger complex localization", JOURNAL OF CELL BIOLOGY, vol. 187, no. 5, novembre 2009 (2009-11), pages 637-653, XP002587552, ISSN: 0021-9525

## Description

La présente invention se situe dans les domaines de la cosmétique et de la pharmacie. La présente invention se rapporte à des composés peptidiques de formule générale (I) R₁ - (AA)ₙ - X₁ - X₂ - Arg - Glu - Met - Asn - Trp - X₃ - (AA)ₚ - R₂, modulateurs de la protéine Survivine, ainsi que leurs utilisations en cosmétique et/ou pharmaceutique afin de (i) prévenir et/ou corriger les signes cutanés du vieillissement et du photo-vieillissement au niveau de la peau et des phanères, (ii) prévenir et/ou limiter la chute des cheveux et/ou stimuler leur croissance, et (iii) protéger la peau contre les agressions extérieures, notamment celles dues aux rayonnements UV.

Le vieillissement correspond à l'ensemble des processus physiologiques et psychologiques qui modifient la structure et les fonctions de l'organisme à partir d'un certain âge. On distingue deux types de vieillissement, d'une part, le vieillissement intrinsèque et, d'autre part, le vieillissement extrinsèque. Le vieillissement intrinsèque est dû à des facteurs génétiques, à des modifications biochimiques qui ont lieu lors d'états de fatigue, de stress, de changements hormonaux comme lors de la grossesse etc. Le vieillissement extrinsèque, quant à lui, est dû à des facteurs environnementaux auxquels est soumis l'organisme tout au long de sa vie, tels que la pollution, le soleil, les maladies etc. Il s'agit d'un processus lent et progressif qui atteint toutes les cellules de l'organisme par différents moyens et se manifeste de différentes manières. Par exemple, au niveau de la peau, l'aspect de celle-ci est modifié par les divers types d'agressions internes ou externes et l'on voit apparaître alors des rides et ridules, des taches d'hyper ou d'hypo-pigmentation, une sécheresse voire une déshydratation de la peau, un amincissement de l'épiderme, une élastose, des imperfections, des taches de vieillesse.... Au niveau des cheveux, le vieillissement se manifeste essentiellement par un blanchiment des cheveux (canitie) mais également par une perte de densité des cheveux, une raréfaction de ceux-ci, conduisant entre autre à une chute plus générale encore appelée alopécie.
Chez les mammifères et particulièrement chez l'homme, la peau et les phanères sont des organes soumis à un renouvellement continuel. Par exemple, le phénomène de desquamation des cellules à la surface de la peau doit être compensé par le renouvellement de l'épiderme assuré par les kératinocytes de la couche basale qui se divisent activement et se différencient en cellules de la couche cornée. Les cheveux ont eux aussi un cycle de renouvellement divisé en 3 phases distinctes appelées anagène, catagène et télogène. C'est lors de la phase télogène qu'un nouveau bulbe pilaire se forme et initie un nouveau cycle de pousse du cheveu. Ces activités de renouvellement, mais aussi de réparation de la peau ou des phanères dans le cas de dommages tels que les rayonnements UV ou les blessures, impliquent l'existence de cellules souches somatiques appelées cellules souches adultes épidermiques dans la peau, et cellules souches adultes folliculaires dans le cas des cheveux. Ces cellules sont assez particulières en ce sens qu'elles présentent des capacités d'auto-renouvellement et de différenciation importantes. Elles ont été identifiées au sein de la couche basale grâce à un ensemble de marqueurs moléculaires tels que la β1-intégrine, l'α6-intégrine, la kératine K15, la kératine K19... La prolifération ainsi que la différenciation cellulaire sont des processus qui sont dépendants du cycle cellulaire. Les mécanismes de contrôle du correct déroulement du cycle cellulaire reposent essentiellement sur un ensemble de structures protéiques complémentaires, comme par exemple le complexe chromosomique passager encore appelé « chromosomal passenger complex » (CPC). Le CPC joue un rôle clé à la fois dans la progression mitotique et aussi lors de l'étape de cytodiérèse. Le CPC est constitué d'un ensemble de 4 protéines qui sont : INCENP (Inner Centromere Protein), Boréaline, la kinase Aurora B et Survivine. La Survivine est exprimée au point G2/M de manière cycle-cellulaire dépendante. Il a été montré que l'invalidation de la Survivine ou de la kinase Aurora B par siRNA induit entre autres des défauts mitotiques majeurs (Lens S. M. et al., EMBO. J. 2003; 22(12): 2934-47). D'autres expériences ont démontré le rôle clé de la Survivine, de son transporteur Crm-1 (ou exportine 1) et de ses partenaires du CPC, au cours du contrôle de la mitose. Enfin, d'autres études, ont permis de classer la Survivine parmi les molécules de la famille des protéines inhibitrices de l'apoptose encore appelées « inhibitor of apoptosis protein » (IAP). En effet, la Survivine protègerait la cellule de l'apoptose en inhibant la voie de signalisation de la caspase-9. Cependant, il semblerait que l'activité mitotique de la Survivine soit prépondérante sur son activité anti-apoptotique. Il a été établi la présence de taux supérieurs de Survivine au sein des cellules souches adultes situées dans la couche basale d'épidermes de sujets jeunes par rapport à des épidermes de sujets âgés. En outre, il a été prouvé que les tissus foetaux contenaient des quantités abondantes de Survivine, alors que celle-ci était quasiment absente dans les tissus différenciés (Adida et al., Am. J. Pathol. 1998 ;152:43-49). C'est ainsi que la corrélation entre la quantité de Survivine dans lesdites cellules souches et le vieillissement de la peau a été faite (Marconi et al., Stem Cells 2007 ;25 :149-55). Et c'est en partant de ces conclusions que, de manière surprenante, la Demanderesse a mise en évidence que des composés peptidiques de formule générale (I) suivante :

R₁-(AA)ₙ-X₁-X₂-Arg-Glu-Met-Asn-Trp-X₃-(AA)p-R₂

étaient de très bons modulateurs de la Survivine et par là même du complexe CPC, et avaient une action significative sur le traitement et/ou la prévention des signes cutanés du vieillissement, sur la protection de la peau contre les agressions extérieures telles que les rayonnements UV, et sur la chute des cheveux.

Jusqu'à présent, aucun composé ayant une action de ce type n'avait été proposé dans le cadre du traitement du vieillissement ou de la chute des cheveux. Certains types de composés peptidiques avaient été proposés en tant que traitements anti-tumoraux par modulation de l'expression de la Survivine (brevets EP1913947, EP1931376). D'autres traitements à l'aide de peptides inhibiteurs de la Survivine ont été divulgués, par exemple, pour inhiber la pousse des poils (brevet EP1841401), ou encore, aider à l'identification de tumeurs (brevet EP2119726) etc. La demande de brevet FR 2 932 086 décrit quant à elle des agents cosmétiques choisis parmi les extraits de *Coleus Forskolii,* de *Lepechinia caulescens,* de *Limnophila conferta,* de *Daniellia oliveri,* de Nostoc commune, de *Scenedesmus dimorphus,* de *Curcuma longa* ou encore de *Crocus sativus* en tant qu'agents activateurs de la survivine pour prévenir ou retarder l'apparition des signes du vieillissement cutané ou activer la pousse des cheveux. Cependant, aucun traitement utilisant des composés modulateurs de la Survivine (et des protéines du CPC) n'a été proposé afin (i) de lutter contre les signes cutanés du vieillissement, (ii) de protéger la peau contre les agressions extérieures, notamment celles dues aux rayonnements UV, et (iii) de limiter la chute des cheveux ou activer leur pousse. Pourtant, il existe un réel besoin de traitements innovateurs dans ce domaine. Ainsi, les composés peptidiques tels que décrits dans la présente invention permettent de proposer un nouveau type de traitement efficace pour lutter contre le vieillissement, en agissant directement sur l'environnement proche des cellules souches adultes épidermiques, afin de protéger celles-ci et leur permettre d'assurer au mieux leur rôle lors des processus de renouvellement et de protection des cellules de la peau. Ces composés peptidiques présentent l'avantage de protéger l'environnement des cellules souches adultes épidermiques et de moduler la quantité de Survivine présente dans ces cellules, sans pour autant accroître leur clonogénicité. De manière générale, les peptides selon l'invention permettent de préserver leur « potentiel » de cellules souches (« Stemness Potential ») en protégeant l'environnement proche qui les entoure et en limitant un possible épuisement desdites cellules. En ayant une action protectrice des cellules souches adultes épidermiques et folliculaires, le composé selon l'invention peut prévenir également la chute des cheveux et peut stimuler la pousse des cheveux.

Par conséquent, la présente invention a pour premier objet un composé peptidique de formule générale suivante (I) :

R₁-(AA)ₙ-X₁-X₂-Arg-Glu-Met-Asn-Trp-X₃-(AA)ₚ-R₂

X₁ représente une leucine, une alanine, ou aucun acide aminé,
X₂ représente une lysine, une proline, ou aucun acide aminé,
X₃ représente une leucine, un acide aspartique, une tyrosine, une phénylalanine, ou aucun acide aminé,
AA représente un acide aminé quelconque, et n et p sont des nombres entiers compris entre 0 et 2,
R₁ représente la fonction amine primaire de l'acide aminé N-terminal, libre ou substituée par un groupement de type acyle possédant une chaîne alkyle de C₁ à C₃₀, saturée ou insaturée, pouvant être choisie parmi un groupement acétyle ou un groupement aromatique, ce dernier pouvant être choisi parmi un groupement de type benzoyle, tosyle, ou benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH2, NHY ou NYY avec Y représentant une chaîne alkyle de C₁ à C₄,
ladite séquence de formule générale (I) étant constituée de 5 à 12 résidus d'acides aminés, ladite séquence de formule générale (I) pouvant comprendre des dérivés ou des substitutions des acides aminés AA, X₁, X₂, ou X₃ par d'autres acides aminés chimiquement équivalents.

La présente invention a pour second objet une composition cosmétique comprenant à titre de principe actif ledit composé peptidique de formule (I).

En outre, la présente invention a pour troisième objet l'utilisation d'une composition cosmétique comprenant ledit composé peptidique de formule (I) pour (i) prévenir et/ou traiter les signes cutanés du vieillissement et du photo-vieillissement et favoriser la régénération tissulaire, (ii) protéger la peau contre les agressions extérieures, notamment celles dues aux rayonnements UV, (iii) rétablir un niveau optimal en Survivine et assurer un meilleur fonctionnement du « Chromosomal Passenger Complex », et (iv) prévenir et/ou limiter la chute des cheveux et/ou stimuler leur croissance.

Enfin, la présente invention a pour quatrième objet un procédé de traitement cosmétique de la peau ou des phanères à traiter, à l'aide de la composition comprenant ledit composé peptidique de formule (I).

Le premier objet de la présente invention se rapporte à un composé peptidique de formule générale (I) :

R₁ - (AA)ₙ -X₁ - X₂ - Arg - Glu - Met - Asn - Trp -X₃ - (AA)ₚ - R₂ (I)

Dans laquelle,
X₁ représente une leucine, une alanine, ou aucun acide aminé,
X₂ représente une lysine, une proline, ou aucun acide aminé,
X₃ représente une leucine, un acide aspartique, une tyrosine, une phénylalanine, ou aucun acide aminé,
AA représente un acide aminé quelconque, et n et p sont des nombres entiers compris entre 0 et 2,
R₁ représente la fonction amine primaire de l'acide aminé N-terminal, libre ou substituée par un groupement de type acyle possédant une chaîne alkyle de C₁ à C₃₀, saturée ou insaturée, pouvant être choisie parmi un groupement acétyle ou un groupement aromatique, ce dernier pouvant être choisi parmi un groupement de type benzoyle, tosyle, ou benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH2, NHY ou NYY avec Y représentant une chaîne alkyle de C₁ à C₄,
ladite séquence de formule générale (I) étant constituée de 5 à 12 résidus d'acides aminés,
ladite séquence de formule générale (I) pouvant comprendre des dérivés ou des substitutions des acides aminés AA, X₁, X₂, ou X₃ par d'autres acides aminés chimiquement équivalents.

Le terme « composé peptidique » ou « peptide » désigne un enchaînement de deux ou plusieurs acides aminés liés entre eux par des liaisons peptidiques ou par des liaisons peptidiques modifiées.
Par « composé peptidique » ou « peptide », il faut entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.
Les acides aminés constituant le composé peptidique selon l'invention peuvent être sous configuration Lévogyre c'est-à-dire L- et/ou Dextrogyre c'est-à-dire D-. Le peptide selon l'invention peut donc être sous forme L-, D- ou DL-.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans les domaines de la cosmétique et de la pharmacie. De préférence, on utilise pour protéger la fonction amine primaire de l'acide aminé N-terminal, une substitution par un groupement R₁ de type acyle possédant une chaîne alkyle de C₁ à C₃₀ saturée ou insaturée, pouvant être choisie parmi un groupement acétyle ou un groupement aromatique. De préférence, on utilise pour protéger la fonction carboxyle de l'acide aminé C-terminal, une substitution par un groupement R₂ de type chaîne alkyle de C₁ à C₃₀, ou un groupement NH₂, NHY ou NYY avec Y représentant une chaîne alkyle de C₁ à C₄.
Le peptide selon l'invention peut être protégé au niveau de l'extrémité N-terminale, C-terminale ou au niveau des deux extrémités.

Dans un premier mode de réalisation préféré, dans la formule (I),
X₁ représente une leucine, ou aucun acide aminé,
X₂ représente une lysine, ou aucun acide aminé,
X₃ représente une tyrosine, ou aucun acide aminé,
Dans AA, les nombres entiers n et p sont égaux à zéro,
R₁ représente la fonction amine primaire de l'acide aminé N-terminal, libre ou substituée par un groupement de type acyle possédant une chaîne alkyle de C₁ à C₃₀, saturée ou insaturée, pouvant être choisie parmi un groupement acétyle ou un groupement aromatique, ce dernier pouvant être choisi parmi un groupement de type benzoyle, tosyle, ou benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH2, NHY ou NYY avec Y représentant une chaîne alkyle de C₁ à C₄,

Dans un second mode de réalisation préféré, le composé peptidique correspond à une des formules suivantes :
(SEQ ID n°1) Ile - Leu - Arg - Glu - Met - Asn - Trp - Gly - NH₂
(SEQ ID n°2) Arg - Glu - Met - Asn - Trp - NH₂
(SEQ ID n°3) Asp - Ala - Arg - Glu - Met - Asn - Trp - Asp - Thr
(SEQ ID n°4) Leu - Arg - Glu - Met - Asn - Trp -Tyr - NH₂
(SEQ ID n°5) Arg - Glu - Met - Asn - Trp -Phe - Met - Val
(SEQ ID n°6) Leu - Lys -Arg - Glu - Met - Asn - Trp - Tyr - NH₂

L'invention concerne aussi des formes homologues de ces séquences. Le terme « homologue » désigne, selon l'invention, toute séquence peptidique identique à au moins 50%, ou de préférence au moins 80%, et encore plus préférentiellement à au moins 90% de ladite séquence peptidique, choisie parmi les séquences SEQ ID n° 1 à SEQ ID n° 6. Par « séquence peptidique identique à au moins X% », on entend désigner un pourcentage d'identité entre les résidus d'acides aminés des deux séquences à comparer, obtenu après l'alignement optimal des deux séquences. L'alignement optimal est obtenu à l'aide d'algorithmes d'homologies locales tels que ceux utilisés par le logiciel informatique BLAST P disponible sur le site NCBI.
Le terme « homologue » peut également désigner un peptide qui diffère de la séquence d'un peptide de séquence SEQ ID n° 1 à SEQ ID n° 6 par la substitution d'acides aminés chimiquement équivalents, c'est-à-dire par la substitution d'un résidu par un autre possédant les mêmes caractéristiques. Ainsi, les substitutions classiques se font entre Ala, Val, Leu et Ile ; entre Ser et Thr ; entre les résidus acides Asp et Glu ; entre Asn et Gln ; et entre les résidus basiques Lys et Arg ; ou entre les résidus aromatiques Phe et Tyr.
Le peptide de formule générale (I) selon l'invention peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.
Le peptide selon l'invention peut être d'origine naturelle ou synthétique. Préférentiellement selon l'invention, le peptide est obtenu par synthèse chimique.
Enfin, le principe actif peut être un peptide unique, un mélange de peptides ou de dérivés peptidiques et/ou constitué de dérivés d'acides aminés.

Le composé peptidique selon l'invention peut être utilisé à titre de médicament.

Selon un mode de réalisation avantageux de l'invention, le composé peptidique selon l'invention est solubilisé dans un ou plusieurs solvants physiologiquement adaptés, classiquement utilisés par l'homme du métier, comme l'eau, le glycérol, l'éthanol, le propanediol, le butylène glycol, le dipropylène glycol, les diglycols éthoxylés ou propoxylés, les polyols cycliques, ou tout mélange de ces solvants.
Selon encore un autre mode de réalisation avantageux de l'invention, le composé peptidique selon l'invention est solubilisé dans un vecteur cosmétique ou pharmaceutique comme les liposomes, ou adsorbé sur des polymères organiques poudreux, des supports minéraux comme les talcs et bentonites, et plus généralement solubilisé dans, ou fixé sur, tout vecteur physiologiquement adapté.

Le composé peptidique selon l'invention est caractérisé en ce qu'il permet de moduler la quantité de protéine Survivine et/ou l'activité du CPC.
On entend par composé peptidique qui « permet de moduler la quantité de protéine Survivine», tout peptide ou dérivé biologiquement actif capable d'augmenter ou diminuer l'activité du complexe CPC et/ou de la Survivine, soit par augmentation ou baisse de la synthèse protéique des protéines du CPC et/ou de la Survivine (par modulation directe ou indirecte de l'expression génique), soit par d'autres processus biologiques tels que la stabilisation ou non des transcrits d'ARN messager.

Le second objet de la présente invention se rapporte à une composition cosmétique comprenant à titre de principe actif ledit composé peptidique de formule générale (I).
De manière préférée, les compositions selon l'invention se présentent sous une forme adaptée à l'application par voie topique comprenant un milieu cosmétiquement acceptable. Par « cosmétiquement acceptable », on entend des milieux qui conviennent à une utilisation en contact avec la peau ou les phanères humains, sans risque de toxicité, d'incompatibilité, d'instabilité, de réponse allergique et autres. Les compositions destinées à être appliquées sur la peau peuvent se présenter sous forme de crème, émulsion huile-dans-eau, ou eau-dans-huile ou émulsion multiple, solution, suspension, microémulsion, gel aqueux ou anhydre, sérum, ou encore de dispersion de vésicules, de patch, de spray, d'onguent, de pommade, de lotion, de colloïde, de lait, de lotion, de stick ou encore de poudre, tous adaptés à une application sur la peau, les lèvres et/ou les phanères.

Préférentiellement, ledit composé peptidique est présent dans la composition à une concentration comprise entre 0,0005 et 500 ppm environ, et préférentiellement à une concentration comprise entre 0,01 et 5 ppm.
Encore plus préférentiellement, la composition selon l'invention contient en outre, au moins un autre principe actif favorisant l'action dudit composé peptidique. On peut citer, de manière non limitative, les classes d'ingrédients suivantes : d'autres agents actifs peptidiques, des extraits de végétaux, des agents cicatrisants, anti-âge, anti-rides, apaisants, anti-radicalaires, anti-UV, des agents stimulant la synthèse de macromolécules dermiques ou le métabolisme énergétique, des agents hydratants, anti-bactériens, antifongiques, anti-inflammatoires, anesthésiques, des agents modulant la différenciation, la pigmentation ou la dépigmentation cutanée, des agents stimulant la pousse des ongles ou des cheveux etc. Préférentiellement, on utilisera un agent présentant une activité dans le domaine des anti-rides, tel qu'un agent anti-radicalaire ou antioxydant, ou un agent stimulant la synthèse de macromolécules dermiques, ou encore un agent stimulant le métabolisme énergétique. Plus particulièrement, le principe actif est choisi parmi les vitamines, les phytostérols, les flavonoïdes, la DHEA et/ou un de ses précurseurs ou un de ses dérivés chimiques ou biologiques, un inhibiteur de métalloproteinase, ou un rétinoïde.
Dans un mode plus particulier de réalisation, la composition selon l'invention comprendra, outre le composé peptidique de formule (I),
- un (ou plusieurs) composé activateur du cytochrome c, et/ou ;
- un (ou plusieurs) composé activateur des aquaporines et/ou ;
- un (ou plusieurs) composé activateur des sirtuines et/ou ;
- un (ou plusieurs) composé qui augmente l'adhésion cellulaire et/ou ;
- un (ou plusieurs) composé qui augmente la production de protéines matricielles type collagène, laminine etc. ;
- un (ou plusieurs) composé modulateur de l'activité du protéasome ;
- un (ou plusieurs) composé modulateur du rythme circadien ;
- un (ou plusieurs) composé modulateur des protéines hsp ;
- un (ou plusieurs) composé qui augmente l'énergie cellulaire ;
- un (ou plusieurs) composé modulateur de la pigmentation ;
- un (ou plusieurs) composé inducteur du Coenzyme Q10 ;
- un (ou plusieurs) composé améliorant la fonction barrière de la peau ;
- un (ou plusieurs) composé protecteur de la mitochondrie.
Lesdits composés ci-dessus peuvent être naturels, comme des hydrolysats peptidiques de végétaux, ou encore d'origine synthétique, comme des composés peptidiques.
En outre, des additifs tels que des solvants, des diluants, des colorants, des filtres solaires, des agents auto-bronzants, des pigments, des charges, des conservateurs, des absorbeurs d'odeurs, des agents épaississants, émulsifiants, humectants, émollients, des parfums, des antioxydants, des agents filmogènes, chélatants, séquestrants, conditionnants...peuvent être ajoutés à la composition.
Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, être compris entre 0,01 et 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés dans une proportion allant de 0,3 à 30 % en poids, par rapport au poids total de la composition.

Un troisième objet de l'invention se rapporte à l'utilisation d'une composition comprenant un composé peptidique selon l'invention afin de rétablir un niveau optimal en Survivine dans les cellules de la peau et des phanères et assurer un meilleur fonctionnement du « Chromosomal Passenger Complex ». On entend par « rétablir un niveau optimal en Survivine », la capacité du composé selon l'invention d'augmenter ou diminuer la quantité de protéine Survivine présente dans les cellules de la peau, notamment les cellules souches adultes épidermiques et folliculaires, afin que celles-ci contrôlent au mieux leurs mitoses, et ainsi répondent au mieux aux besoins de renouvellement des cellules épidermiques.

Un autre objet de l'invention concerne l'utilisation d'une composition cosmétique comprenant ledit composé peptidique et un milieu cosmétiquement acceptable pour prévenir et/ou traiter les signes cutanés du vieillissement et du photo-vieillissement, et favoriser la régénération tissulaire. Les « signes cutanés du vieillissement » incluent, mais ne se limitent pas à, toutes les manifestations visibles sur la peau causées par le vieillissement. On entend, en particulier, les rides, les rides profondes et grossières, les ridules, les crevasses, le relâchement des tissus cutanés et sous-cutanés, la perte de l'élasticité cutanée et l'atonie, la perte de fermeté et de tonicité, et l'atrophie dermique. En outre, on entend aussi par « signes cutanés du vieillissement », les pores élargis, les imperfections, la décoloration, les taches de vieillesse, les kératoses, la perte de collagène, et autres changements du derme et de l'épiderme, mais également toutes modifications de l'aspect extérieur de la peau et des phanères dues au vieillissement comme, par exemple, les rugosités superficielles de la couche cornée, mais également toute modification interne de la peau qui ne se traduit pas systématiquement par un aspect extérieur modifié comme, par exemple, l'amincissement du derme. Par « photo-vieillissement », on entend le vieillissement prématuré de la peau causé par l'exposition prolongée et cumulative au soleil.
On entend par « régénération tissulaire », la reconstitution d'un tissu à partir d'une ou plusieurs cellules. Le processus de régénération tissulaire intervient notamment lors de blessures et de la cicatrisation. Le composé peptidique selon l'invention peut favoriser ce processus de régénération via notamment la protection de l'environnement des cellules souches adultes épidermiques et folliculaires. Des tests menés en *in vitro* ont montré une augmentation de l'expression des protéines qui forment l'environnement proche propre aux cellules souches. Les protéines dont l'expression était augmentée sont la β1-intégrine, l'a6-intégrine, la kératine K15, ainsi que la kératine K19 qui est une kératine très spécifique des cellules souches adultes situées dans le bulbe pileux.

Un autre objet de l'invention porte sur l'utilisation d'une composition selon l'invention pour protéger la peau contre les agressions extérieures, notamment celles dues aux rayonnements UV. Par « agressions extérieures », on entend les agressions que peut produire l'environnement. A titre d'exemple, on peut citer des agressions telles que la pollution, les UV, ou encore les produits à caractère irritant tels que les tensioactifs, les conservateurs ou les parfums, les agressions mécaniques, telles que les abrasions, le rasage ou l'épilation. La sécheresse de l'atmosphère est également une cause importante d'agression cutanée. Tous ces phénomènes entraînent une réduction de l'épaisseur de la couche basale. Celle-ci étant moins épaisse et contenant moins de cellules souches adultes épidermiques, la régénération de la peau s'effectue de moins en moins bien et le vieillissement s'accélère. Des tests menés en *in vitro* ont montré que l'application d'une composition comprenant un composé peptidique selon l'invention permettait de limiter l'impact des agressions des rayonnements UV sur les cellules qui composent la couche basale, d'où le rôle protecteur de ces composés.

Dans un mode particulièrement préféré, l'invention porte sur l'utilisation d'une composition comprenant ledit composé peptidique pour prévenir et/ou limiter la chute des cheveux, et/ou stimuler leur croissance. Des tests menés sur des biopsies de peau et des follicules pileux ont montré que l'actif selon l'invention avait une action protectrice sur les follicules lors, notamment, d'expositions à des rayonnements UVA et UVB. Le composé selon l'invention possède donc une action protectrice des cellules folliculaires contre les agressions extérieures, et notamment contre les rayonnements UV.
Enfin, un dernier objet de la présente invention se rapporte à un procédé de traitement cosmétique caractérisé en ce que l'on applique topiquement sur la peau ou les phanères à traiter, une composition comprenant une quantité efficace de composé peptidique selon l'invention pour prévenir et/ou traiter les signes cutanés du vieillissement et du photo-vieillissement et favoriser la régénération tissulaire.

Les exemples suivants décrivent et démontrent l'efficacité de composés peptidiques tels que décrits selon l'invention mais ne doivent pas être interprétés comme une limitation de la présente invention.

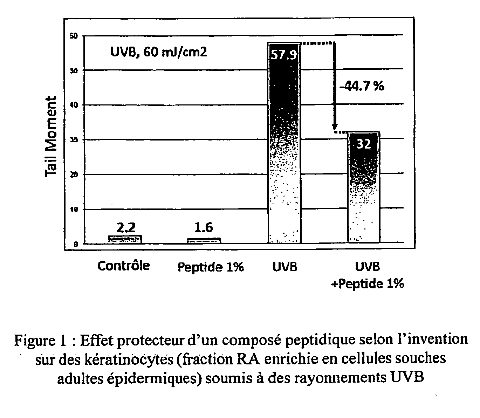

La figure 1 est un histogramme présentant les résultats d'un test comètes réalisé sur une fraction cellulaire enrichie en cellules souches adultes épidermiques.

### Exemple 1 : Préparation des fractions cellulaires et quantification de la protéine Survivine

Afin d'étudier le rôle des protéines du CPC dans le maintien et la protection des cellules souches adultes épidermiques lors de différents stress, plusieurs fractions de cellules primaires d'épidermes humains sont préparées. Le fractionnement est basé sur le temps d'adhésion des cellules sur du Collagène IV : en moins de 20 minutes, adhésion rapide (fraction RA), en moins de 5 heures, adhésion lente n°1 (fraction SA1), en moins de 16 heures, adhésion lente n°2 (fraction SA2), et finalement les kératinocytes non adhérents (fractions NA). La fraction cellulaire RA contient les cellules qui adhèrent le plus rapidement donc les moins différenciées et, par conséquent, RA est la fraction enrichie en cellules souches adultes épidermiques. En revanche, les fractions SA1, SA2 et NA sont celles qui contiennent respectivement de moins en moins de cellules souches. Par la suite, les niveaux de protéine Survivine ont été caractérisés par Western Blot.

### Résultats :

A l'issue de cette caractérisation, on constate que la Survivine est surexprimée dans les cellules RA et que les niveaux d'expression diminuent progressivement dans les cellules SA1, SA2 pour finalement être quasi nuls dans les cellules NA.

### Exemple 2 : Mise en évidence de l'effet activateur du peptide SEO ID n°2 sur l'expression de la protéine Survivine dans les fractions cellulaires RA et SA1

Une étude de l'effet modulateur du peptide SEQ ID n°2, a été réalisée en évaluant l'expression de la protéine Survivine en Western Blot sur les fractions cellulaires RA et SA1 en culture. La technique de Western Blot est une méthode semi-quantitative classique qui permet d'apprécier le taux de protéines dans les cellules.

### Protocole

Les fractions RA et SA1 sont cultivées dans des boîtes de diamètres 100 mm à 37°C dans une atmosphère humidifiée contenant 5 % de CO₂ pendant 48 heures en présence ou non du composé, dilué 3 % (à partir d'une solution 10⁻⁴ M). Les cellules sont rincées puis détachées du support à l'aide d'un tampon d'extraction (25 mM Tris-HCl pH 7.6, 150 mM NaCl, 1 % NP-40, 1 % Sodium deoxycholate and 0.1 % SDS) en présence d'un cocktail d'inhibiteurs de protéases (Halt protease inhibitor PIERCE). Les protéines ainsi extraites, sont centrifugées à 4°C à 10000 rpm pendant 10 minutes avant d'être dosées par le kit de dosages des protéines BCA (Pierce). Les lysats cellulaires sont mélangés à un tampon de dénaturation et soumis à une électrophorèse SDS-PAGE. Le gel utilisé est un Nupage 4-12 % (invitrogen). Les protéines sont ensuite transférées sur une membrane avec la machine à transfert Iblot (Invitrogen). Les membranes sont saturées dans un mélange TBS-lait 5 %/0.1 % tween 20 pendant 2 heures à température ambiante, puis incubées à 4°C toute la nuit avec un anticorps primaire anti-Survivine au 1/1000^{ième} (Cell signaling #2808- lapin) suivie de 3 lavages en TBS, 0,1 % tween puis d'une incubation avec un anticorps secondaire anti-lapin couplé à la peroxydase dilué au 1/5000^{ième} (Immunotech). La révélation est effectuée par un substrat chimio- luminescent. L'évaluation quantitative des protéines présentes dans les cellules est effectuée grâce au logiciel chemiimager (Alpha Innotech Corporation USA). La quantité des protéines est exprimée en pourcentage d'intensité lumineuse comparée à la condition contrôle qui n'a pas reçu de traitement.

### Résultats :

Le peptide SEQ ID n°2 permet d'augmenter l'expression de la protéine Survivine dans les fractions très riches en cellules souches adultes épidermiques (RA) ainsi que dans des fractions moins riches en cellules souches adultes épidermiques (SA1).

### Exemple 3 : Test comètes sur la fraction cellulaire RA

Le test comètes est un test qui permet de quantifier les dommages causés à l'ADN au niveau cellulaire.
Dans ce but, afin de vérifier l'effet protecteur d'un composé peptidique selon l'invention, des cellules de type RA sont mises en culture pendant 24 heures avec le composé peptidique de séquence SEQ ID n° 2 à une concentration de 1 %, puis irradiées avec des rayonnements UVB à 60 mJ/cm². Une condition contrôle est réalisée sans actif peptidique. Les cellules sont ensuite trypsinées de leur support, puis centrifugées à 900 tour/min pendant 5 minutes afin de les concentrer et les dénombrer.
Un nombre défini de cellules (25 000 cellules) est ensuite inclus dans un gel d'agarose Low Melting à 0.75 %, puis déposé sur une lame de verre préalablement recouverte d'agarose à 1 %. Les lames sont ensuite immergées dans une solution de lyse pendant une heure 30 à 4 °C, puis dans une solution alcaline pendant 20 minutes à 4 °C. Les cellules sont ainsi lysées et l'ADN dénaturé. Les lames sont plongées dans une solution d'électrophorèse avant d'appliquer un champ électrique (20 V - 250 mA). L'ADN ainsi dénaturé est soumis à une migration au sein du gel d'agarose à 4 °C. L'application d'un colorant fluorescent de l'ADN sur les lames (l'iodure de propidium à 2 µg/ml) permet d'observer au microscope l'ADN apparaissant sous forme de comètes dans le cas où il a été endommagé.
Un logiciel de quantification permet de déterminer le Tail moment moyen appliqué à chaque condition testée. Ce paramètre renseigne sur le niveau d'endommagement de l'ADN.

### Résultats :

Les résultats sont présentés dans la figure 1. Le Tail Moment diminue de 44,7 % lorsque le peptide est appliqué, c'est-à-dire que les cellules ont subies moins de dommages que dans la condition contrôle avec rayonnements UVB. Les résultats confirment bien l'effet protecteur du composé peptidique de séquence SEQ ID n°2 sur les cellules issues des fractions enrichies en cellules souches adultes épidermiques (RA).

### Exemple 4 : Test MITOSOX

Le Mitosox est un réactif commercialisé par Invitrogen (molecular probes M36008). Il est utilisé lors d'un stress oxydatif UV et H₂O₂. Il permet de mesurer la production mitochondriale de Radicaux libres (ROS) dans les cellules en imagerie microscopique. Plus particulièrement, le Mitosox est spécifique de l'anion superoxyde qui est la forme prédominante de ROS.
Le Mitosox s'applique sur cellule vivante : il est perméant à la membrane, et une fois à l'intérieur de la cellule, il diffuse rapidement et de façon spécifique dans la mitochondrie. Une fois dans la mitochondrie, le Mitosox est oxydé par l'anion superoxyde en un composé qui présente une fluorescence rouge. Ce réactif est facilement oxydé par l'anion superoxyde mais pas par les autres radicaux libres, ni par les espèces réactives de l'azote (NOS).

### Protocole :

Des kératinocytes RA et TOTAL (TOTAL signifie une fraction cellulaire complète) sont traités trois fois par jour avec le composé peptidique de séquence SEQ ID n° 3 à des concentrations de 1 % et 3 % pendant 48 heures.
Dans un second temps, les cellules sont stressées :
- Soit par un stress à l'aide d'une solution d'H₂O₂ à 5 mM pendant 30 minutes
- Soit par des rayonnements UVB à 100 mJ/cm²
Après le stress, les cellules sont rincées puis remises dans un milieu adapté pendant une heure à 37 °C dans un incubateur de culture. Il s'en suit deux lavages à l'aide de milieu de Hanks, l'application de la solution de Mitosox pendant 5 minutes, et enfin de nouveaux lavages à l'aide du même milieu. Les cellules sont fixées avec du formaldéhyde pendant 10 minutes et du DAPI est appliqué pendant 5 minutes, avant deux rinçages à l'aide de PBS. Les résultats sont lus par lecture en microscopie à fluorescence.

### Résultats :

En microscopie à fluorescence, on constate que dans la condition contrôle, le nombre de cellules présentant des dommages aussi bien dus aux rayonnements UVB, qu'à l' H2O2, est très important. En effet, la fluorescence est très importante dans cette condition ce qui indique une forte production de radicaux libres. Par contre, lorsque le peptide est ajouté aux cellules avant l'exposition au stress, on constate que le nombre de cellules fluorescentes est extrêmement faible. Ceci implique donc que le peptide a protégé les cellules contre la production excessive de radicaux libres.

### Exemple 5: Action du composé peptidique SEQ ID n°2 sur des follicules pileux maintenus en culture et marquage de la kératine K19

### Culture des follicules pileux et inclusions :

Les follicules pileux sont prélevés d'une biopsie à l'aide d'un scalpel et de fines pinces, puis mis en culture dans des plaques 24 puits avec du milieu William's E en présence d'antibiotiques (Primocine 0,2 %), d'insuline 10 µg/ml, d'Hydrocortisone 10 ng/ml et de L-Glutamine 2 mmol/L.
Les follicules sont traités ou non avec le composé peptidique SEQ ID n° 2 à 1 % dans le milieu William's, puis sont incubés pendant 24 heures. L'irradiation UV est ensuite appliquée (UVA 5 J/cm² et UVB 200 mJ/cm²), puis les follicules sont remis en culture pendant 24 heures. A la fin de l'expérience, les follicules sont inclus en OCT puis congelés à - 196 °C (azote liquide). Les follicules sont ensuite placés dans un cryostat afin de réaliser des coupes de 6 µm à une température de - 23 °C. Les lames contenant les coupes sont mises dans un bain d'acétone pendant 10 minutes (fixation) et sont ensuite rincées.

### Immunomarquage de la Kératine K19 :

Les coupes fixées sont rincées au PBS une fois pendant 5 minutes, puis entourées de Pappen. Chaque coupe est incubée avec 50 µl de BSA 5 % pendant 30 minutes. Puis, 50 µl d'anticorps primaire anti-Cytokératine K19 (Progen, Mouse monoclonal) dilué au 1/50^{ième} sont ajoutés et incubés pendant une heure sous agitation en chambre humide. Après rinçage avec du PBS pendant 30 minutes, 50 µl d'un anticorps secondaire marqué à la fluorescence sont ajoutés et laissés durant une heure à l'obscurité sous agitation en chambre humide. Les lames sont ensuite rincées dans le PBS, montées entre lame et lamelle dans du fluoromount G, et l'observation s'effectue au microscope à épifluoresence.

### Résultats :

L'irradiation des follicules par les UV a entrainé une baisse de - 48 % de l'intensité de fluorescence de la Kératine K19 dans lesdits follicules pileux (quantification par analyse d'images). Les follicules ayant reçu le traitement par le composé peptidique SEQ ID n°2 avant irradiation montrent des valeurs de fluorescence de la Kératine K19 de + 102,7 %. Ceci signifie que le composé peptidique selon l'invention a permis de prévenir et d'empêcher l'apparition de dommages au niveau des cellules, notamment au niveau de la production de Kératine K19.

### Exemple 6 : Compositions

### 1. Composition d'une crème solaire

| Noms commerciaux | Noms INCI | % massique |
|---|---|---|
| PHASE A | | |
| Eau déminéralisée | Aqua (Water) | qsp |
| Pemulen TR1 | Acrylates/C 10-30 Alkyl | 0,40 |
| | Acrylate Crosspolymer | |
| Glycerine | Glycerin | 3,00 |
| Nipastat Sodium | Sodium Methylparaben (and) | 0,15 |
| | Sodium Ethylparaben (and) | |
| | Sodium Butyl paraben (and) | |
| | Sodium Propylparaben (and) | |
| | Sodium Isobutylparaben | |

| PHASE B | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |

| Noms commerciaux | Noms INCI | % massique |
|---|---|---|
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Peptide SEQ ID n°2 | | 3 ppm |
| Parfum | Parfum (Fragrance) | qsp |
| Colorant | | qsp |

### 2. Sérum pour la pousse des cheveux

Disperser le Natrosol 250HHR et le Disodium EDTA dans l'eau sous agitation. Chauffer à 50-60 °C et agiter jusqu'à obtenir un aspect uniforme. Ajouter le Styleze^{®} CC-10 et agiter jusqu'à obtenir un aspect uniforme. Laisser refroidir à température ambiante et ajouter les ingrédients dans l'ordre de la liste en agitant jusqu'à obtenir un aspect uniforme entre chacun d'eux.

| **Nom INCl** | **Nom commercial** | **% massique** | **Fournisseur** |
|---|---|---|---|
| Eau | | Q.S. | |
| Hydroxyethylcellulose | Natrosol 250HHR | 0,35 | Hercules/Aqualon |
| Disodium EDTA | Dissolvine NA-2S | 0,05 | Akzo Nobel |
| VP/DMAPA Acrylates Copolymères | Styleze® CC-10 | 5,00 | ISP |
| Quatemium-26 | Ceraphyl® 65 | 1,00 | ISP |
| Panthénol | Ritapan DL | 0,15 | RITA |
| Propylène Glycol Urée Diazolidinyle Iodopropynyl Butylcarbamate | Liquid Germall® Plus | 0,50 | ISP |
| Peptide SEQ ID N°5 | | 2 ppm | ISP |
| Total | | 100,00 | |

## Revendications

1. Composé peptidique de formule générale suivante (I) :
R₁-(AA)ₙ-X₁-X₂-Arg-Glu-Met-Asn-Trp-X₃-(AA)ₚ-R₂
Dans laquelle,
X₁ représente une leucine, une alanine, ou aucun acide aminé,
X₂ représente une lysine, une proline, ou aucun acide aminé,
X₃ représente une leucine, un acide aspartique, une tyrosine, une phénylalanine, ou aucun acide aminé,
AA représente un acide aminé quelconque, et n et p sont des nombres entiers compris entre 0 et 2,
R₁ représente la fonction amine primaire de l'acide aminé N-terminal, libre ou substituée par un groupement de type acyle possédant une chaîne alkyle de C₁ à C₃₀, saturée ou insaturée, pouvant être choisie parmi un groupement acétyle ou un groupement aromatique, ce dernier pouvant être choisi parmi un groupement de type benzoyle, tosyle, ou benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH2, NHY ou NYY avec Y représentant une chaîne alkyle de C₁ à C₄,
ladite séquence de formule générale (I) étant constituée de 5 à 12 résidus d'acides aminés, ladite séquence de formule générale (I) pouvant comprendre des dérivés ou des substitutions des acides aminés AA, X₁, X₂, ou X₃ par d'autres acides aminés chimiquement équivalents.

2. Composé peptidique selon la revendication 1 **caractérisé en ce que** dans la formule générale (I)
X₁ représente une leucine, ou aucun acide aminé,
X₂ représente une lysine, ou aucun acide aminé,
X₃ représente une tyrosine, ou aucun acide aminé,
Dans AA, les nombres entiers n et p sont égaux à zéro,
R₁ représente la fonction amine primaire de l'acide aminé N-terminal, libre ou substituée par un groupement de type acyle possédant une chaîne alkyle de C₁ à C₃₀, saturée ou insaturée, pouvant être choisie parmi un groupement acétyle ou un groupement aromatique, ce dernier pouvant être choisi parmi un groupement de type benzoyle, tosyle, ou benzyloxycarbonyle,
R₂ représente le groupe hydroxyle de la fonction carboxyle de l'acide aminé C-terminal, libre ou substitué par un groupement pouvant être choisi parmi une chaîne alkyle de C₁ à C₃₀, ou un groupement NH2, NHY ou NYY avec Y représentant une chaîne alkyle de C₁ à C₄,

3. Composé peptidique selon la revendication 1 ou 2, **caractérisé en ce qu'**il correspond à une des formules suivantes :
(SEQ ID n°1) Ile-Leu-Arg-Glu-Met-Asn-Trp-Gly-NH₂
(SEQ ID n°2) Arg-Glu-Met-Asn-Trp-NH₂
(SEQ ID n°3) Asp-Ala-Arg-Glu-Met-Asn-Trp-Asp-Thr
(SEQ ID n°4) Leu-Arg-Glu-Met-Asn-Trp-Tyr-NH₂
(SEQ ID n°5) Arg-Glu-Met-Asn-Trp-Phe-Met-Val-NH₂
(SEQ ID n°6) Leu-Lys-Arg-Glu-Met-Asn-Trp-Tyr-NH₂

4. Composé peptidique selon l'une des revendications précédentes utilisé à titre de médicament.

5. Composition cosmétique comprenant à titre de principe actif ledit composé peptidique selon l'une des revendications 1 à 3.

6. Composition cosmétique selon la revendication 5, **caractérisée en ce qu'**elle se présente sous une forme adaptée à l'application par voie topique comprenant un milieu cosmétiquement acceptable.

7. Composition selon l'une des revendications 5 ou 6, **caractérisée en ce que** ledit composé peptidique est présent dans la composition à une concentration comprise entre 0,0005 et 500 ppm environ, et préférentiellement à une concentration comprise entre 0,01 et 5 ppm.

8. Composition selon l'une quelconque des revendications 5 à 7, **caractérisée en ce qu'**elle contient en outre, au moins un autre principe actif favorisant l'action dudit composé peptidique.

9. Composition selon la revendication 8, **caractérisée en ce que** ledit principe actif est un agent présentant une activité dans le domaine des antirides tel qu'un agent anti-radicalaire ou antioxydant, ou un agent stimulant la synthèse de macromolécules dermiques, ou encore un agent stimulant le métabolisme énergétique.

10. Utilisation d'une composition cosmétique comprenant ledit composé peptidique tel que défini dans l'une des revendications 1 à 3 et un milieu cosmétiquement acceptable pour rétablir un niveau optimal en Survivine dans les cellules de la peau et des phanères et assurer un meilleur fonctionnement du «Chromosomal Passenger Complex».

11. Utilisation d'une composition cosmétique comprenant ledit composé peptidique tel que défini dans l'une des revendications 1 à 3 et un milieu cosmétiquement acceptable pour prévenir et/ou traiter les signes cutanés du vieillissement et du photo-vieillissement et favoriser la régénération tissulaire.

12. Composition cosmétique comprenant ledit composé peptidique tel que défini dans l'une des revendications 1 à 3 et un milieu cosmétiquement acceptable destinée à être utilisée pour protéger la peau contre les agressions extérieurs, notamment celles dues aux rayonnements UV.

13. Utilisation selon la revendication 10 pour prévenir et/ou limiter la chute des cheveux et/ou stimuler leur croissance.

14. Utilisation selon la revendication précédente pour stimuler la pousse des cils.

15. Procédé de traitement cosmétique **caractérisé en ce que** l'on applique topiquement sur la peau ou les phanères à traiter, le matin et/ou le soir, une composition comprenant une quantité efficace de composé peptidique tel que défini dans l'une des revendications 1 à 3, pour prévenir et/ou traiter les signes cutanés du vieillissement et du photo-vieillissement, favoriser la régénération tissulaire, prévenir et/ou limiter la chute des cheveux et/ou activer leur croissance.

## Patentansprüche

1. Peptidzusammensetzung mit der folgenden allgemeinen Formel (I):
R1 - (AA)ₙ - X₁ - X₂ - Arg - Glu - Met - Asn - Trp - X₃ -(AA)ₚ - R₂.
wobei:
X₁ ein Leucin, ein Alanin oder keine Aminosäure darstellt.
X₂ ein Leucin, ein Prolin, oder keine Aminosäure darstellt.
X₃ ein Leucin, eine Asparaginsäure, ein Tyrosin, ein Phenylalanin oder keine Aminosäure darstellt.
AA irgendeine Aminosäure darstellt und n und p ganze Zahlen zwischen 0 und 2 sind,
R₁ die primäre Amionfunktion der N-terminalen Aminosäure darstellt, frei oder substituiert durch eine Gruppe vom Typ Acyl, umfassend eine Alkylkette von C₁ bis C₃₀, gesättigt oder ungesättigt, die ausgewählt sein kann aus einer Acetylgruppe oder einer aromatischen Gruppe, wobei die letztere ausgewählt werden kann aus einer Gruppe vom Typ Benzoyl, Tosyl oder Benzyloxycarbonyl,
R₂ die Hydroxylgruppe der Carboxylfunktion der C-terminalen Aminosäure darstellt, frei oder substituiert durch eine Gruppe, die aus einer Alkylkette von C₁ bis C₃₀ ausgewählt werden kann, oder einer Gruppe NH2, NHY oder NYY, wobei Y eine Alkylkette von C₁ bis C₄ darstellt,
wobei die Sequenz mit der allgemeinen Formel (1) aus 5 bis 12 Rückständen von Aminosäuren besteht, wobei die Sequenz mit der allgemeinen Formel (I) Derivate oder Substitutionen der Aminosäuren AA, X₁, X₂ oder X₃ durch andere chemisch gleichwertige Aminosäuren umfassen kann.

2. Peptidzusammensetzung nach Anspruch 1, **dadurch gekennzeichnet, dass** in der allgemeinen Formel (I)
X₁ ein Leucin, oder keine Aminosäure darstellt.
X₂ ein Lysin oder keine Aminosäure darstellt.
X₃ ein Tyrosin oder keine Aminosäure darstellt.
In AA die ganzen Zahlen n und p gleich null sind,
R₁ die primäre Amionfunktion der N-terminalen Aminosäure darstellt, frei oder substituiert durch eine Gruppe vom Typ Acyl, umfassend eine Alkylkette von C₁ bis C₃₀, gesättigt oder ungesättigt, die ausgewählt sein kann aus einer Acetylgruppe oder einer aromatischen Gruppe, wobei diese Letztere ausgewählt werden kann aus einer Gruppe vom Typ Benzoyl, Tosyl oder Benzyloxycarbonyl,
R₂ die Hydroxylgruppe der Carboxylfunktion der C-terminalen Aminosäure darstellt, frei oder substituiert durch eine Gruppe, die aus einer Alkylkette von C₁ bis C₃₀ oder einer NH2-, NHY- oder NYY-Gruppe gewählt werden kann, wobei Y eine Alkylkette von C₁ bis C₄ darstellt.

3. Verfahren nach Anspruch 1 oder 2, **dadurch gekennzeichnet, dass** sie einer der folgenden Formeln entspricht:
(SEQ ID n°1) Ile-Leu-Arg-Glu-Met-Asn-Trp - Gly - NH₂
(SEQ ID n°2) Arg - Glu - Met - Asn - Trp - NH₂
(SEQ ID n°3) Asp - Ala - Arg - Glu - Met - Asn - Trp - Asp - Thr
(SEQ ID n°4) Leu - Arg - Glu - Met - Asn - Trp-. Tyr - NH₂
(SEQ ID n°5) Arg - Glu - Met - Asn - Trp - Phe - Met - Val - NH₂
(SEQ ID n°6) Leu - Lys - Arg - Glu - Met - Asn - Trp - Tyr - NH₂

4. Peptidzusammensetzung nach einem der vorhergehenden Ansprüche, die als Arzneimittel verwendet wird.

5. Kosmetische Zusammensetzung, umfassend als Wirkstoff die Peptidzusammensetzung nach einem der Ansprüche 1 bis 3.

6. Kosmetische Zusammensetzung nach Anspruch 5, **dadurch gekennzeichnet, dass** sie in einer Form vorliegt, die an die Anwendung auf topischem Weg angepasst ist, umfassend eine kosmetisch akzeptables Medium.

7. Zusammensetzung nach einem der Ansprüche 5 oder 6, **dadurch gekennzeichnet, dass** die Peptidzusammensetzung in der Zusammensetzung in einer Konzentration vorhanden ist, die zwischen ungefähr 0,0005 und 500 ppm liegt und vorzugsweise in einer Konzentration, die zwischen 0,01 und 5 ppm liegt.

8. Zusammensetzung nach einem der Ansprüche 5 bis 7, **dadurch gekennzeichnet, dass** sie außerdem mindestens einen weiteren Wirkstoff umfasst, der die Wirkung der Peptidzusammensetzung begünstigt.

9. Zusammensetzung nach Anspruch 8, **dadurch gekennzeichnet, dass** der Wirkstoff ein Mittel ist, das eine Aktivität in der Domäne der Antifalten-Zusammensetzungen aufweist, wie z.B. ein Antiradikal oder ein Antioxidationsmittel oder ein Mittel, das die Synthese von Hautmakromolekülen stimuliert, oder auch ein Mittel, das den Energiestoffwechsel stimuliert.

10. Verwendung einer kosmetischen Zusammensetzung, umfassend die Peptidzusammensetzung, wie in einem der Ansprühe 1 bis 3 definiert, und ein kosmetisch akzeptables Milieu, um ein optimales Niveau an Survivin in den Zellen der Haut und der Hautanhangsgebilde wieder herzustellen und eine bessere Funktion des "Chromosomal Passenger Complex" sicherzustellen.

11. Verwendung einer kosmetischen Zusammensetzung, umfassend die Peptidzusammensetzung, wie in einem der Ansprühe 1 bis 3 definiert, und ein kosmetisch akzeptables Milieu, um den Hautanzeichen der Alterung und der Lichtalterung vorzubeugen und/oder diese zu behandeln und die Geweberegeneration zu begünstigen.

12. Kosmetische Zusammensetzung, umfassend die Peptidzusammensetzung, wie in einem der Ansprüche 1 bis 3 definiert, und ein kosmetisch akzeptables Milieu, dazu ausgelegt, um verwendet zu werden, um die Haut gegen äußere Aggressionen zu schützen, insbesondere diejenigen augrund von UV-Strahlungen.

13. Verwendung nach Anspruch 10, um dem Haarausfall vorzubeugen und/oder ihn zu begrenzen und/oder um das Haarwachstum zu stimulieren.

14. Verwendung nach dem vorhergehenden Anspruch, um das Wachstum der Augenwimpern zu stimulieren.

15. Verfahren zur kosmetischen Behandlung, **dadurch gekennzeichnet, dass** morgens und/oder abends topisch auf die zu behandelnde(n) Haut/Hautanhangsgebilde eine Zusammensetzung aufgebracht wird, die eine wirksame Menge der Peptidzusammensetzung, wie in einem der Ansprüche 1 bis 3 definiert, umfasst, um den Hautanzeichen der Alterung und der Lichtalterung vorzubeugen und/oder diese zu behandeln und die Geweberegeneration zu begünstigen, dem Haarausfall vorzubeugen und/oder ihn zu begrenzen und/oder das Wachstum der Haare zu begünstigen.

## Claims

1. Peptide compound according to the following general formula (I) :
R₁-(AA)ₙ-X₁-X₂-Arg-Glu-Met-Asn-Trp-X₃-(AA)ₚ-R₂
Wherein,
X₁ represents a leucine, an alanine, or no amino acid,
X₂ represents a lysine, a proline, or no amino acid,
X₃ represents a leucine, an aspartic acid, a tyrosine, a phenylalanine, or no amino acid,
AA represents any amino acid, and n and p are integers between 0 and 2,
R₁ represents the primary amine function of the N-terminal amino acid, which is free or substituted with an acyl type group having a saturated or unsaturated C₁ to C₃₀ alkyl chain, suitable for being chosen from an acetyl group or an aromatic group, the latter being suitable for being chosen from a benzoyl, tosyl, or benzylcarbonyl group,
R₂ represents the hydroxyl group of the carboxyl function of the C-terminal amino acid, which is free or substituted with a group suitable for being chosen from a C₁ to C₃₀ alkyl chain, or an NH2, NHY or NYY group where Y represents a C₁ to C₄ alkyl chain,
said sequence according to general formula (I) consisting of 5 to 12 amino acid residues,
said sequence according to general formula (I) optionally comprising derivatives or substitutions of the amino acids AA, X₁, X₂, or X₃ with other chemical equivalent amino acids.

2. Peptide compound according to claim 1, **characterized in that** the general formula (I)
X₁ represents a leucine, or no amino acid,
X₂ represents a lysine, or no amino acid,
X₃ represents a tyrosine, or no amino acid,
In AA, the integers n and p are equal to zero.
R₁ represents the primary amine function of the N-terminal amino acid, which is free or substituted with an acyl type group having a saturated or unsaturated C₁ to C₃₀ alkyl chain, suitable for being chosen from an acetyl group or an aromatic group, the latter being suitable for being chosen from a benzoyl, tosyl, or benzylcarbonyl group,
R₂ represents the hydroxyl group of the carboxyl function of the C-terminal amino acid, which is free or substituted with a group suitable for being chosen from a C₁ to C₃₀ alkyl chain, or an NH2, NHY or NYY group where Y represents a C₁ to C₄ alkyl chain.

3. Peptide compound according to claim 1 or 2, **characterized in that** it corresponds to one of the following formulas:
(SEQ ID No. 1) Ile-Leu-Arg-Glu-Met-Asn-Trp-Gly-NH₂
(SEQ ID No. 2) Arg-Glu-Met-Asn-Trp-NH₂
(SEQ ID No. 3) Asp-Ala-Arg-Glu-Met-Asn-Trp-Asp-Thr
(SEQ ID No. 4) Leu-Arg-Glu-Met-Asn-Trp-Tyr-NH₂
(SEQ ID No. 5) Arg-Glu-Met-Asn-Trp-Phe-Met-Val-NH₂
(SEQ ID No. 6) Leu-Lys-Arg-Glu-Met-Asn-Trp-Tyr-NH₂

4. Peptide compound according to any of the above claims used as a medicinal product.

5. Cosmetic composition comprising, as an active ingredient, said peptide compound according to any of claims 1 to 3.

6. Cosmetic composition according to claim 5, **characterised in that** it is presented in a form suitable for topical application comprising a cosmetically acceptable medium.

7. Composition according to any of claims 5 or 6, **characterised in that** said peptide compound is present in the composition at a concentration between approximately 0.0005 and 500 ppm, and preferentially at a concentration between 0.01 and 5 ppm.

8. Composition according to any of claims 5 to 7, **characterised in that** it further contains at least one further active ingredient promoting the action of said peptide compound.

9. Composition according to claim 8, **characterised in that** said active ingredient is an agent having an activity in the field of anti-wrinkle treatments such as an anti-radical or antioxidant agent, or an agent stimulating the synthesis of dermal macromolecules, or an agent stimulating the energy metabolism.

10. Use of a cosmetic composition comprising said peptide compound as defined in any of claims 1 to 3 and a cosmetically acceptable medium for restoring an optimal level of Survivin in skin and skin appendage cells and ensuring an enhanced "Chromosomal Passenger Complex" function.

11. Use of a cosmetic composition comprising said peptide compound as defined in any of claims 1 to 3 and a cosmetically acceptable medium for preventing and/or treating skin signs of ageing and photoageing and promoting tissue regeneration.

12. Cosmetic composition comprising said peptide compound as defined in any of claims 1 to 3 and a cosmetically acceptable medium intended to be used for protecting the skin against external attacks, particularly those due to UV rays.

13. Use according to claim 10 for preventing and/or limiting hair loss and/or stimulating hair growth.

14. Use according to the above claim for stimulating eyelash growth.

15. Cosmetic treatment method **characterised in that** a composition comprising an effective quantity of peptide compound as defined in any of claims 1 to 3 is applied topically onto the skin and skin appendages to be treated, to prevent and/or treat skin signs of ageing and photoageing, promote tissue regeneration, prevent and/or limit hair loss and/or activate hair growth.


### SEQUENCE LISTING

<110> ISP Investments Inc
<120> NOUVEAUX PEPTIDES ANTI-AGE MODULATEURS DE LA SURVIVINE ET COMPOSITIONS LES COMPRENANT
<130> Bv PCT 10-136_148
<150> FR 10 00134
   <151> 2010-01-14
<160> 6
<170> PatentIn version 3.3
<210> 1
   <211> 8
   <212> PRT
   <213> Artificial
<220>
   <223> Synthetic peptide
<220>
   <221> MOD_RES
   <222> (8)..(8)
   <223> AMIDATION
<400> 1
   Ile Leu Arg Glu Met Asn Trp Gly
   1               5
<210> 2
   <211> 5
   <212> PRT
   <213> Artificial
<220>
   <223> Synthetic peptide
<220>
   <221> MOD_RES
   <222> (5)..(5)
   <223> AMIDATION
<400> 2
   Arg Glu Met Asn Trp
   1               5
<210> 3
   <211> 9
   <212> PRT
   <213> Artificial
<220>
   <223> Synthetic peptide
<400> 3
   Asp Ala Arg Glu Met Asn Trp Asp Thr
   1               5
<210> 4
   <211> 7
   <212> PRT
   <213> Artificial
<220>
   <223> Synthetic peptide
<220>
   <221> MOD_RES
   <222> (7)..(7)
   <223> AMIDATION
<400> 4
   Leu Arg Glu Met Asn Trp Tyr
   1               5
<210> 5
   <211> 8
   <212> PRT
   <213> Artificial
<220>
   <223> Synthetic peptide
<220>
   <221> MOD_RES
   <222> (8)..(8)
   <223> AMIDATION
<400> 5
   Arg Glu Met Asn Trp Phe Met Val
   1               5
<210> 6
   <211> 8
   <212> PRT
   <213> Artificial
<220>
   <223> Synthetic peptide
<220>
   <221> MOD_RES
   <222> (8)..(8)
   <223> AMIDATION
<400> 6
   Leu Lys Arg Glu Met Asn Trp Tyr
   1 5